# EUROPEAN PATENT APPLICATION

(11) **EP 4 599 786 A1**
(43) Date of publication of application: **13.08.2025**
(21) Application number: 25157521.3
(22) Date of filing: 12.02.2025
(51) Int. Cl.: A61B 46/20, A61B 46/27

(54) **A SURGICAL DRAPE**

(30) Priority: 12.02.2024 US 202418439586
(71) Applicant: Medenvision, 2260 Westerlo (BE)
(72) Inventor: DEBOECK, Pieter, 3390 Tielt-Winge (BE); VAN LIMBERGEN, Filip, 2235 Hulshout (BE); FOULON, Wouter, 3210 Lubbeek (BE); CORTEN, Kristoff, 3201 Wolfsdonk (BE); GOYENS, Floris, 2020 Antwerpen (BE)
(74) Representative: Gevers Patents

(57) **Abstract**

The present invention relates to a surgical drape (35, 135) for shoulder surgery. The surgical drape (35, 135) comprises a main covering layer (10, 110) having a front surface (18, 118) and a back surface (38, 138), an absorbing layer (2, 102) applied to the front surface wherein a first cut is made through the absorbing layer and the main covering layer, the cut being configured that the drape can move over an arm through the cut, a limb sleeve (5, 105) having a closed end and an open end wherein the limb sleeve is assembled to the main covering layer at the open end and the cut aligned such that when the drape is moved over the arm through the cut, the arm is covered by the limb sleeve, and a tourniquet element (12, 121) having an elastic opening configured to move over an arm with a circumferential pressure on the arm, wherein perforations are provided in the limb sleeve for tearing off part of the limb sleeve and wherein the tourniquet element is assembled in the limb sleeve at a position between the open end of the limb sleeve and the perforations.

## Description

### FIELD OF THE INVENTION

The present invention relates to a surgical drape and to methods for using a surgical drape for preparing a patient for surgical procedure.

### BACKGROUND OF THE INVENTION

Surgical drapes are known. They are often not easy to use and require multiple surgical drapes especially for surgical procedures at difficult areas such as for example the shoulder.

Especially shoulder surgery requires today a lot of time to prepare the patient for the surgical procedure. The patient is covered by multiple sterile drapes until only the area needed of the surgical procedure is not covered.

There is a need for a surgical drape which allows easier preparation for a shoulder surgical procedure. Next to a surgical drape, there is a further need for a method to prepare the patient in an easier, more predictable and faster way for surgery on the shoulder.

### SUMMARY OF THE INVENTION

It is an aim of the present invention to provide a surgical drape which overcomes at least some of the above mentioned problems.

This aim is achieved according to the invention with a surgical drape for shoulder surgery, the surgical drape, comprising a main covering layer having a front surface and a back surface, an absorbing layer applied to the front surface wherein a first cut is made through the absorbing layer and the main covering layer, the cut being configured that the drape can move over an arm through the cut, a limb sleeve having a closed end and an open end wherein the limb sleeve is assembled to the main covering layer at the open end and the cut aligned such that when the drape is moved over the arm through the cut, the arm is covered by the limb sleeve, and a tourniquet element comprising an elastic opening configured to move over an arm and a connection edge assembled in the limb sleeve at a first distance from the open end, wherein perforations are provided in the limb sleeve for tearing off part of the limb sleeve at a second distance from the open end, and wherein the first distance is lower than the second distance such that the tourniquet element is connected in the limb sleeve at a position between the open end of the limb sleeve and the perforations.

It is an advantage of the surgical drape according to the invention that it enables preparing a patient with the surgical drape in a fast way with a minimum risk of sterilization errors because the drape can cover the patient in only a few standardized steps and the perforations allow to create an accessible area without touching the accessible area with hands. It is a further advantage of the present invention, that fewer steps, less time, and less manpower is required in order to cover a patient in a sterile manner, without compromising the freedom of the surgeon to tailor the surgical window to the requirements of the shoulder surgery and patient characteristics.

In some embodiments of the invention, the surgical drape further comprises a reinforcement element, wherein the reinforcement element has a second cut, the second cut being configured such that the drape can move over an arm through the second cut and wherein the reinforcement element is applied to the back surface of the main covering layer with the second cut aligned to the first cut such that the drape can move over an arm through the first cut and the second cut.

The reinforcement element reinforces not only the opening, it also protects the connection of the limb sleeve to the main covering layer resulting in a more reliable drape.

In some embodiments of the invention, the limb sleeve has a larger width at the open end than at the closed end. In a particular embodiment, the width of the limb sleeve at the open end is between 35cm and 45cm, preferably 40cm. In a particular embodiment, the width of the limb sleeve at the closed end is between 25cm and 35cm, preferably 30cm. It is an advantage of these dimensions that the limb sleeve is also suitable for patients with large arms, while less material is used than if the width was the same at both ends of the limb sleeve.

In some embodiments of the invention, the surgical drape, in particular the absorbing layer, further comprises an attachment element adjacent to the first cut. The attachment element is configured for attaching a part of the limb sleeve, in particular the part between the open end of the limb sleeve and the perforations, to the absorbing layer. It is an advantage that after a part of the limb sleeve has been torn off from the rest of the surgical drape, the remaining part of the limb sleeve can be attached to the rest of the surgical drape, it cannot accidentally fall down into the surgical field, and obscure the view. In this manner, the remaining part of the limb sleeve remains above the elastic opening of the tourniquet element.

In some embodiments of the invention, the perforations are at a distance between 5cm and 9cm from the open edge of the limb sleeve, preferably between 6cm and 8cm, and most preferred at 7cm from the open edge of the limb sleeve.

The location of the perforation with respect to the open end has the advantage that there is sufficient space for assembling the tourniquet element between the open edge and the perforations and at the same time have the perforations close enough to the open end to be able to tear off close to the shoulder.

In some embodiments of the invention, the perforations are provided circumferential around the limb sleeve.

This has the advantage that it is easy to tear off part of the limb sleeve.

In some embodiments of the invention, the surgical drape further comprises a means for indicating the location of the perforations, wherein the means for indicating the location of the perforations is provided on the limb sleeve.

This has the advantage that the user knows well where to tear off part of limb sleeve saving time.

In some embodiments of the invention, the means for indicating the location of the perforations is glued on the limb sleeve.

This has the advantage that it is a trusted way of providing something.

In some embodiments of the invention, the limb sleeve comprises a sleeve starting layer which is folded in the length direction and glued on both sides of the folded parts to form the sleeve with a closed end and an open end, wherein the folded parts are glued up to a first distance from the open end, and wherein the perforations are at a second distance from the open end, wherein the second distance is larger than or equal to the first distance.

This has the advantage that part of the limb sleeve can be easier torn off at the perforations and that the part of the limb sleeve that remains in the drape is still glued together at the sides.

In some embodiments of the invention, the limb sleeve is glued to the main covering layer.

This has the advantage that gluing is a reliable way of assembling preventing the limb sleeve from loosening.

In some embodiments of the invention, the tourniquet element is glued in the limb sleeve.

This has the advantage that gluing is a reliable way of assembling preventing the tourniquet element from loosening.

It is a further aim of the present invention to provide a method of sterile covering of a patient for shoulder surgery which overcomes at least some of the above mentioned problems.

This aim is achieved according to the invention with a method of sterile covering of a patient for shoulder surgery using a surgical drape wherein the surgical drape is folded such that the limb sleeve can unfold without unfolding the main covering layer, the method comprising
- moving the surgical drape through the first cut over an arm of a patient causing that the arm is covered by the limb sleeve,
- unfolding the main covering layer over the patient,
- tearing off part of the limb sleeve at the perforations,
- moving the torn off part of the limb sleeve over the arm away from the shoulder to create an accessible area of desired size for the surgery,
- fixing the position of torn off part of the limb sleeve on the arm when the desired size for the accessible area is reached.

It is an advantage of the method of sterile covering of a patient for shoulder surgery that the patient is sterile covered in a fast way with a minimum risk of sterilization errors because the drape can cover the patient in only a few steps and the perforations allow to create an accessible area without touching the accessible area with hands. It is a further advantage of the present invention, that fewer steps, less time, and less manpower is required in order to cover a patient in a sterile manner.

In some embodiments of the invention, the fixing of the torn off part of the limb sleeve is executed by applying a tape on the torn off part of the limb sleeve.

This has the advantage that this can be executed fast in a reliable way and that it can be executed with minimum risk of sterilization errors.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiments described hereinafter.

### BRIEF DESCRIPTION OF THE DRAWINGS

FIG. 1 illustrates layers of a surgical drape according to an embodiment of the invention.
FIG. 2 illustrates a second part of the main covering layer according to an embodiment of the invention.
FIG. 3 illustrates a sleeve starting layer to form a limb sleeve according to an embodiment of the invention.
FIG. 4 illustrates a limb sleeve according to an embodiment of the invention.
FIG. 5 illustrates a tourniquet element positioned in a limb sleeve according to an embodiment of the invention.
FIG. 6 illustrates a starting layer to form a tourniquet element according to an embodiment of the invention.
FIG. 7 illustrates a reinforcement element to apply to a main covering layer at the opening for a limb according to an embodiment of the invention.
FIG. 8 illustrates a reinforcement element positioned on a main covering layer according to an embodiment of the invention.
FIG. 9 illustrates a surgical drape according to an embodiment of the invention.
FIG. 10 illustrates layers of a surgical drape according to an alternative embodiment of the invention.
FIG. 11 illustrates a second part of the main covering layer according to an alternative embodiment of the invention.
FIG. 12 illustrates a sleeve starting layer to form a limb sleeve according to an alternative embodiment of the invention.
FIG. 13 illustrates a limb sleeve according to an alternative embodiment of the invention.
FIG. 14 illustrates a tourniquet element positioned in a limb sleeve according to an alternative embodiment of the invention.
FIG. 15 illustrates a starting layer to form a tourniquet element according to an alternative embodiment of the invention.
FIG. 16 illustrates a reinforcement element to apply to a main covering layer at the opening for a limb according to an alternative embodiment of the invention.
FIG. 17 illustrates a reinforcement element positioned on a main covering layer according to an alternative embodiment of the invention.
FIG. 18 illustrates a surgical drape according to an alternative embodiment of the invention.

The drawings are only schematic and are non-limiting. In the drawings, the size of some of the elements may be exaggerated and not drawn on scale for illustrative purposes. Further, relative dimensions may not correspond to actual reductions to practice of the invention.

Any reference signs in the claims shall not be construed as limiting the scope.

In the different drawings, the same reference signs refer to the same or analogous elements.

### DETAILED DESCRIPTION OF THE INVENTION

The present invention will be described with respect to particular embodiments and with reference to certain drawings but the invention is not limited thereto but only by the claims.

The terms "up", "down", "high", "low", "horizontal", "vertical", "top", "bottom", "side" and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

### Surgical drape

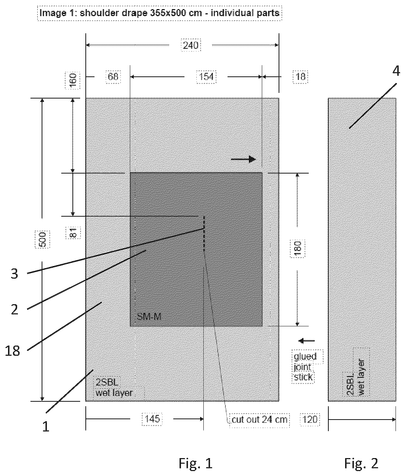

In a first aspect, the present invention relates to a surgical drape for sterile covering a patient for a shoulder surgery procedure. By way of illustration and not limited thereto, embodiments of the surgical drape are illustrated in Figures 1 to 5, 9, 10 to 14, and 18.

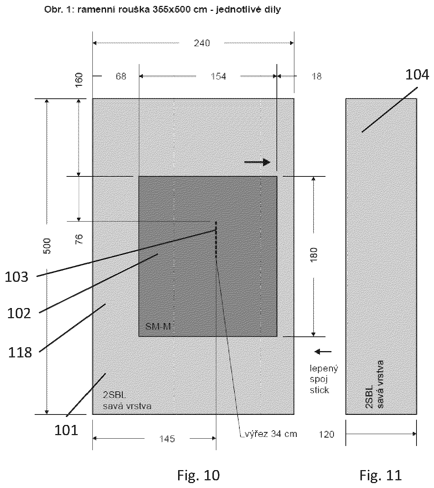

Figures 1 to 5, and 10 to 14 illustrate different parts which gradually create the surgical drape during manufacturing. Figures 1 and 10 illustrate a relatively large first layer 1, 101 from 2SBL material wherein 2SBL is a 2-ply laminated material comprising a lamination of hydrophilic spunbond and PE foil. This first layer has in the embodiment of Figures 1 and 10 dimensions 240cm x 500cm. In alternative embodiments these dimensions may be smaller or larger. They can be smaller or larger in one direction or in both directions. In alternative embodiments the first layer may be made of another material known in the art suitable for a sterile drape.

Figures 1 and 10 further illustrate a second layer 2, 102 which is glued on top of the first layer 1, 101. In the embodiment of Figures 1 and 10, the second layer 2, 102 has smaller dimensions than the first layer 1, 101. The second layer 2, 102 has dimensions of 154cm x 180cm. In alternative embodiments the dimensions of the second layer 2, 102 may be smaller or larger, and may have the same or different dimensions than the first layer 1, 101. The second layer 2, 102 is made of SM-M material. SM-M material is an absorbent material. The second layer 2, 102 is in the context of the current application also called the absorbing layer 2, 102. In alternative embodiments the second layer may be made of another absorbent material known in the art suitable for a sterile drape.

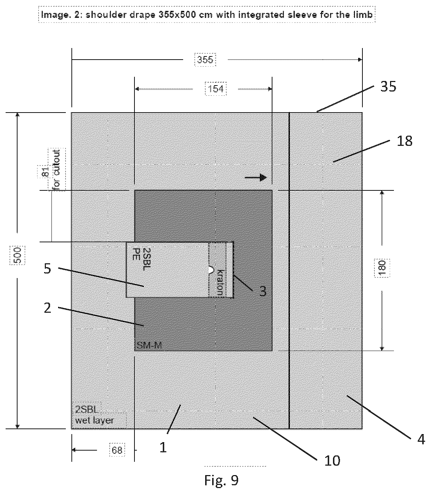

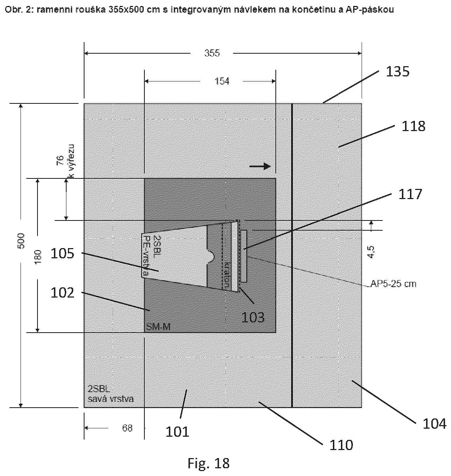

After the second layer 2, 102 is glued on the first layer 1, 101, a cut 3, 103 is made through both layers, thus through the second layer 2, 102 and the first layer 1, 101. In the embodiment of Figures 1 and 10, the cut 3, 103 is made over a straight line. In the embodiment of Figure 1, the cut 3has a length of 24cm. In the embodiment of Figure 10, the cut 103 has a length of 34cm. In other alternative embodiments the shape of the cut 3, 103 may be different and/or the length of the cut 3, 103 may be smaller or larger. The cut 3, 103 is formed such that a sleeve 5, 105 can be positioned in the cut 3, 103. Therefore, the cut 3, 103 is configured such that a sleeve 5, 105 can be positioned and connected in or on the cut 3, 103, as illustrated in Figures 9 and 18.

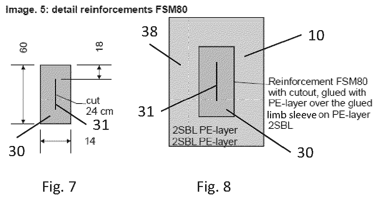

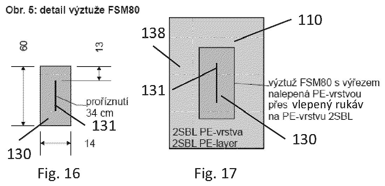

Figures 2 and 11 show a second part 4, 104 made of the first layer material. The second part 4, 104 is connected to the first layer 1, 101 to create a larger first layer 10. The resulting larger first layer 10, 110 is of the same material as the first layer. In the embodiment of Figures 1-2 and 10-11, this is of 2SBL material. In the embodiment of Figures 1-2 and 10-11, the width of the first layer 1, 101 is made larger than 240cm by gluing a second part 4, 104 with a width of 120cm to it forming a larger first layer 10, 110 with a width of 355cm. 5cm of the combined width is used to glue forming an overlap of 2,5cm. In alternative embodiments, the first layer 1, 101 may not need an extension with a second part 4, 104 to increase the width. In still an alternative embodiment the first layer is built up with more than two parts. The resulting first layer, independent if it is made out of a single unglued part or out of multiple parts glued together, is also called for the remaining of the description the main covering layer 10, 110. The main covering layer 10, 110 is shown in Figures 9 and 18. The main covering layer 10, 110 has a front surface 18, 118 and a back surface 38, 138. The back surface 38, 138 is shown on Figures 8 and 17.

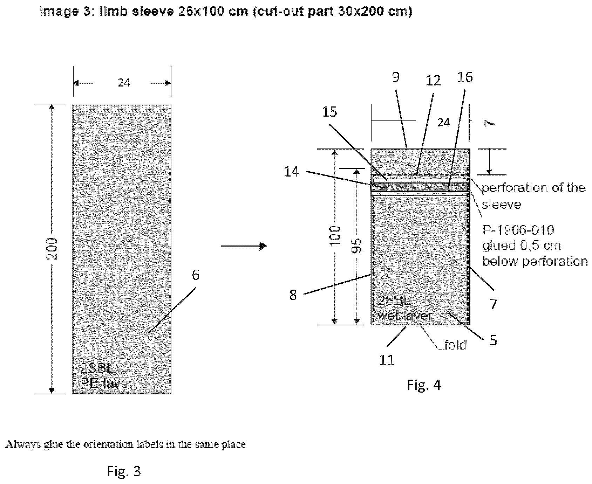

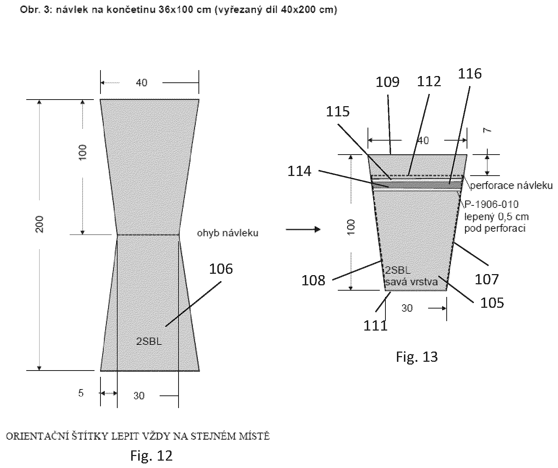

Figure 3 illustrates a sleeve starting layer 6 for a limb sleeve 5 shown in Figure 4. The sleeve starting layer 6 has in the embodiment of Figure 3 a width of 24cm and a length of 200cm. Figures 12 and 13 illustrate an alternative embodiment of the sleeve starting layer 106 and the limb sleeve 105. The limb sleeve 105 has a trapezoid shape. In particular, the width of the limb sleeve 105 at the open edge 109 is larger than the width of the limb sleeve at the folded edge 111. The sleeve starting layer 106 has in the embodiment of Figures 12 and 13 a width of 40cm at the open edge 109, a width of 30cm at the folded edge 111, and a length of 200cm. In alternative embodiments, the width at the open edge is between 30cm and 70cm, preferably between 30cm and 50cm, more preferably between 35cm and 45cm, and most preferably at 40cm. In alternative embodiments, the width at the closed edge is between 20cm and 60cm, preferably between 20cm and 40cm, more preferably between 25cm and 35cm, and most preferably at 30cm. The sleeve starting layer 6, 106 is made of 2SBL material, the same material as the main covering layer 10, 110. In other alternative embodiments, the sleeve starting layer 6, 106 may be larger or smaller. It can be larger or smaller in one or two directions or both. In alternative embodiments the sleeve starting layer 6, 106 may be made of another material known in the art suitable for a sterile drape.

The sleeve starting layer 6 is subsequently folded in the length direction such that the folded sleeve starting layer 6 has still the same width, 24cm in the embodiment of Figure 4, and half of the length of before folding. Likewise, in the embodiment of figure 13, the folded sleeve starting layer 106 has a width of 40cm at the open edge 109, a width of 30cm at the folded edge 111, and half of the length of before folding. In the embodiment of Figures 3-4 and 12-13 the folded length is 100cm. The sides 7, 107 and 8, 108 of the two folded parts are subsequently glued up to 5cm of the open edge 9, 109 i.e. the edge opposite to the folded edge 11, 111. Therefore, in the embodiment where the folded length is 100cm, the sides are glued over a length of 95cm. In alternative embodiments, the sides of the folded sleeve starting layer 6, 106 are glued up to a distance from the edge between 2cm and 8cm, preferably between 3cm and 7cm, more preferably between 4cm and 6cm, and most preferably at 5cm. Further, the folded sleeve starting layer 6, 106 is perforated over the width creating perforations 12, 112 in both folded parts over the width. The perforations 12, 112 are in the embodiments of Figures 4 and 13 at 7cm from the open edge 9, 109. In alternative embodiments the perforations are at a distance between 5cm and 9cm from the open edge, preferably between 6cm and 8cm, and most preferred at 7cm. In an alternative embodiment the perforations 12, 112 are at a distance from the open edge 9, 109 which is larger than or equal to the distance from the open edge that the sides are not glued. In alternative embodiments, the perforations are not forming a line but any other shape.

In alternative embodiments, the distance where the gluing stops from the open edge 9, 109 at the left side 8, 108 is different from the distance where the gluing stops from the open edge 9, 109 at the right side 7, 107.

In some embodiments, the limb sleeve 5, 105 may have a label 14, 114 for providing an indication to a user where the perforations 12, 112 are in the limb sleeve. In alternative embodiments, the label 14, 114 may be any other means known in the art for providing an indication to a user. In the embodiments of Figures 4 and 13, the label 14, 114 is glued on the folded sleeve starting layer 6, 106 over the width of the sleeve starting layer 6, 106. The label 14, 114 has two layers, a base label layer 15, 115 and a central label layer 16, 116. The base label layer 15, 115 may have a height which is larger than the central label layer 16, 116 as in the embodiments of Figures 4 and 13. In alternative embodiments, the height of the base label layer 15, 115 and the central label layer 16, 116 may be the same. In still alternative embodiments, the height of the base label layer 15, 115 may be smaller than the central label layer 16, 116. In the embodiments of Figures 4 and 13, the label 14, 114 is glued on the sleeve starting layer 6, 106 at a distance of 0,5cm of the perforations. In alternative embodiments the label 14, 114 may be positioned at another distance from the perforations, over the perforations or at the other side of the perforations. In alternative embodiments the label 14, 114 has a different shape or may be printed on the sleeve starting layer. In some embodiments, the label 14, 114 is at both sides of the folded sleeve starting layer. In other embodiments, the label 14, 114 is at one side of the folded sleeve starting layer. In alternative embodiments the label 14, 114 is at a distance from the perforations between 0cm and 5cm, preferably between 0cm and 3cm, more preferably between 0cm and 1cm, and most preferably at 0,5cm.

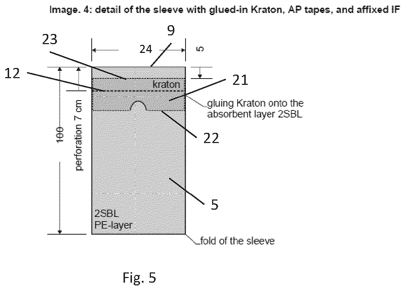

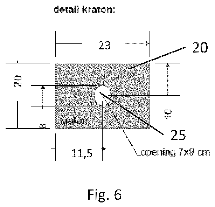

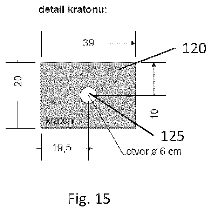

Figures 6 and 15 illustrates an elastomer layer 20, 120 which is prepared for gluing in the limb sleeve 5, 105. In the embodiment of Figures 5-6 and 14-15 the elastomer layer 20, 120 is made of Kraton. Kraton is a high-performance elastomer and a synthetic replacement for rubber. It has especially a high flexibility and traction. In alternative embodiments, the elastomer layer 20, 120 may be from any other flexible material known in the art and useful for a sterile drape. In the embodiment of Figure 6, the elastomer layer 20 has a width of 23cm and a height of 20cm. In the middle of the elastomer layer 20, an opening 25 of 7cm x 9cm is provided. In the alternative embodiment of Figure 15, the elastomer layer 120 has a width of 39cm and a height of 20cm. In the middle of the elastomer layer 120, an opening 125 with a diameter of 6cm is provided. In other alternative embodiments these dimensions may be smaller or larger. They can be smaller or larger in one direction or in both directions. The width of the elastomer layer 20, 120 is in all embodiments suitable for assembling the elastomer layer in the limb sleeve 5, 105.

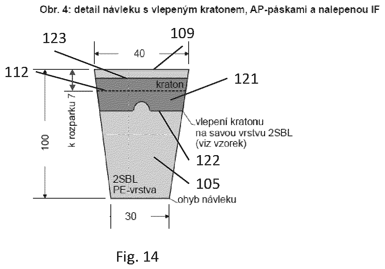

The elastomer layer 20, 120 is folded in the height direction forming a tourniquet element 21, 121 which is assembled in the limb sleeve 5, 105 as illustrated in Figures 5 and 14. The tourniquet element 21, 121 has a folded edge 22, 122 through the opening 25, 125. Opposite to the folded edge 22, 122 is a connection edge 23, 123. The tourniquet element 21, 121 is assembled in the limb sleeve 5, 105 at the connection edge 23, 123. In the embodiments of Figures 5 and 14, the tourniquet element 21, 121 is glued to the sleeve starting layer 6, 106 of the limb sleeve 5, 105. In alternative embodiments, the tourniquet element 21, 121 is assembled in the limb sleeve 5, 105 by any other connection means known in the art.

The positioning of the tourniquet element 21 in the limb sleeve 5 is in the embodiment of Figure 5 such that the connection edge 23 is at 5cm from the open edge 9 of the limb sleeve and at 2 cm from the perforations 12. In alternative embodiments, the distance between the connection edge 23, 123 and the open edge 9, 109 is smaller or larger. In some embodiments, the connection edge 23, 123 is positioned between the open edge 9, 109 and the perforations 12, 112. In alternative embodiments, the tourniquet element 21, 121 is not connected in the sleeve but connected to the main covering layer 10, 110.

The limb sleeve 5, 105 is assembled to the main covering layer 10, 110 on which the absorbing layer 2,102 is already glued as illustrated in Figures 9 and 18. In alternative embodiments the absorbing layer 2, 102 may be assembled after the limb sleeve 5, 105 is assembled to the main covering layer 10, 110. In the embodiment of Figures 9 and 18, the limb sleeve 5,105 is positioned in the cut 3, 103 and an end portion of the two folded parts of the limb sleeve is glued to the back surface of the main covering layer 10, 110. The resulting surgical drape 35, 135 is a drape comprising a limb sleeve 5, 105, a tourniquet element 21, 121, a main covering layer 10, 110 and an absorbing layer 2, 102 wherein part of the limb sleeve can be torn off from the rest of the surgical drape 35, 135.

Figures 7 and 8, 16 and 17 illustrates a reinforcement element 30, 130 which is in some embodiments of the invention glued on the back surface of the main covering layer 10, 110 after the limb sleeve 5, 105 is assembled to the main covering layer 10, 110. In the embodiments of Figures 7 and 16, the reinforcement element 30, 130 has a width of 14cm and a length of 60cm. In alternative embodiments these dimensions may be smaller or larger. They can be smaller or larger in one direction or in both directions. The reinforcement element 30, 130 may be made of any material known in the art suitable for reinforcing a surgical drape. The reinforcement layer further has a cut 31, 131 creating an opening which, in use, provides an opening for the patient to enter a limb through the opening in the limb sleeve.

As illustrated in Figures 8 and 17, the reinforcement element 30, 130 is glued on the main covering layer 10, 110 over the glued limb sleeve portions which are already glued to the back surface of the main covering layer.

Figure 18 illustrates an attachment element 117 which is in some embodiments of the invention comprised on the absorbing layer 2, 102 adjacent to the first cut 3, 103. The attachment element 117 is configured for attaching a part of the limb sleeve 5, 105 to the absorbing layer 2, 102. In particular, the attachment element 117 is configured for attaching a part of the limb sleeve 5, 105 between the open edge 109 and the perforations 112 to the absorbing layer 2, 102. The attachment element 117 may be a double-sided tape, a hook-and-loop connection such as Velcro, or any other material known in the art which is suitable for attaching surgical drape materials to each other. In a particular embodiment, the attachment element 117 is a straight double-sided tape. For the double-sided tape, one side of the tape is attached to the absorbing layer 2, 102; the other side of the tape is covered by a removable protective layer. The removable protective layer can be removed to expose the glue layer such that the part of the limb sleeve 5, 105 can be glued to the absorbing layer 2, 102. In the embodiment of Figure 18, the attachment element has a length of 25cm. In alternative embodiments, the attachment element 117 may be longer or shorter. In alternative embodiments, the attachment element 117 may have a length of between 5cm and 50cm, preferably between 10cm and 40cm, more preferably between 20cm and 30cm, and most preferably at 5cm. The dimensions of the attachment element are in all embodiments suitable for secure attachment of a part of the limb sleeve 5, 105 to the absorbing layer 2, 102.

**Method of preparing a patient for a shoulder surgery using a surgical drape according to an embodiment of the invention.**

In a second aspect, the present invention relates to a method of preparing a patient for shoulder surgery using a surgical drape 35, 135 according to an embodiment.

The surgical drape 35 made according to one of the embodiments described above is folded to distribute and to use in an easy way. The surgical drape is folded with the opening on the back of the main covering layer accessible when folded and such that the limb sleeve 5, 105, which is also folded, can unfold when a patient is entering a limb through the opening on the back of the main covering layer 10, 110.

The folded surgical drape is positioned with the opening at the end of the limb that need to enter the limb sleeve for surgery. The surgical drape can subsequently be moved over the limb causing the limb sleeve to unfold. Once the limb is positioned in the limb sleeve, the main covering layer 10, 110 is unfolded over the patient. The limb sleeve is now torn such that part of the limb sleeve from the perforations to the end is disconnected from the surgical drape. The disconnected limb sleeve part is moved over the limb such that a part of the limb becomes accessible. When the limb is sufficiently accessible, the disconnected limb sleeve part is hold in that position and a tape is applied on the disconnected limb sleeve part to keep it in the desired position. As a result, an accessible area is created with at the one side the tourniquet element 21, 121 creating a circumferential pressure on the limb and on the other side the disconnected limb sleeve part kept in place by a tape. In some further embodiments of the invention, the remaining part of the limb sleeve which remained connected to the surgical drape is subsequently moved away from the accessible area and attached to the surgical drape. This attachment can for example be done be done by means of attachment element 117, or by means of tape. The advantage of this method is that a patient can be prepared for surgery in a minimum amount of steps. And only the surgical drape made as a single part and tape is needed for the preparation.

In some further embodiments of the invention, the surgical drape 35, 135 may be combined with one or more additional surgical drapes into a surgical drape set. Such additional surgical drapes may be positioned before or after the surgical drape 35, 135. In a particular embodiment, the surgical drape set comprises a surgical drape 35, 135 and a rectangular, preferably a square-shaped, additional surgical drape. The additional surgical drape may have a side length of between 50cm and 150cm, preferably between 60cm and 100cm, most preferably 75cm. Preferably, the additional surgical drape comprises an adhesive side. In a particular embodiment, the method of preparing a patient for shoulder surgery comprises positioning the additional surgical drape followed by positioning of the surgical drape 35, 135. The additional surgical drape is positioned such that it covers the head of the patient. The adhesive side of the additional surgical drape is glued to the patient between the head of the patient and the shoulder of the patient. The advantage of this method is that sterility errors during positioning and unfolding of the surgical drape 35, 135 are further minimized, because the head of the patient is in advance covered by a sterile additional surgical drape.

## Claims

1. A surgical drape (35, 135) for sterile covering of a patient for a shoulder surgery, comprising
- a main covering layer (10, 110) having a front surface (18, 118) and a back surface (38, 138),
- an absorbing layer (2, 102) applied to the front surface (18, 118) wherein a first cut is made through the absorbing layer and the main covering layer, the cut being configured that the drape can move over an arm through the cut,
- a limb sleeve (5, 105) having a closed end and an open end wherein the limb sleeve is assembled to the main covering layer at the open end and the cut aligned such that when the drape is moved over the arm through the cut, the arm is covered by the limb sleeve, and
- a tourniquet element (21, 121) having an elastic opening configured to move over an arm and a connection edge assembled in the limb sleeve at a first distance from the open end,
wherein perforations are provided in the limb sleeve for tearing off part of the limb sleeve at a second distance from the open end, and
wherein first distance is lower than the second distance such that the tourniquet element is connected in the limb sleeve at a position between the open end of the limb sleeve and the perforations.

2. The surgical drape according to claim 1, wherein the surgical drape further comprises a reinforcement element (30, 130), wherein the reinforcement element has a second cut, the second cut being configured that the drape can move over an arm through the second cut and wherein the reinforcement element is applied to the back surface of the main covering layer with the second cut aligned to the first cut such that the drape can move over an arm through the first cut and the second cut.

3. The surgical drape according to claim 1 or 2, wherein the limb sleeve (5, 105) has a larger width at the open end than at the closed end.

4. The surgical drape according to anyone of the preceding claims, wherein the absorbing layer 2, 102 adjacent to the first cut 3, 103 further comprises an attachment element (117) which is configured for attaching a part of the limb sleeve 5, 105, in particular a part of the limb sleeve between the open end of the limb sleeve and the perforations, to the absorbing layer 2, 102.

5. The surgical drape according to anyone of the preceding claims, wherein the perforations are at a distance between 5cm and 9cm from the open edge of the limb sleeve, preferably between 6cm and 8cm, and most preferred at 7cm from the open edge of the limb sleeve.

6. The surgical drape according to anyone of the preceding claims, wherein the perforations are provided circumferential around the limb sleeve.

7. The surgical drape according to anyone of the preceding claims, further comprising a means for indicating the location of the perforations, wherein the means for indicating the location of the perforations is provided on the limb sleeve.

8. The surgical drape according to claim 7, wherein the means for indicating the location of the perforations is glued on the limb sleeve.

9. The surgical drape according to anyone of the preceding claims, wherein the limb sleeve comprises a sleeve starting layer which is folded in the length direction and glued on both sides of the folded parts to form the sleeve with a closed end and an open end, wherein the folded parts are glued up to a first distance from the open end, and wherein the perforations are at a second distance from the open end wherein the second distance is larger than or equal to the first distance.

10. The surgical drape according to anyone of the preceding claims, wherein the limb sleeve is glued to the main covering layer.

11. The surgical drape according to anyone of the preceding claims, wherein the tourniquet element is glued in the limb sleeve.

12. A method of sterile covering of a patient for shoulder surgery using a surgical drape according to any one of the preceding claims 1 to 11 wherein the surgical drape is folded such that the limb sleeve can unfold without unfolding the main covering layer, comprising
- moving the surgical drape through the first cut over an arm of a patient causing that the arm is covered by the limb sleeve,
- unfolding the main covering layer over the patient,
- tearing off part of the limb sleeve at the perforations,
- moving the torn off part of the limb sleeve over the arm away from the shoulder to create an accessible area of desired size for the surgery,
- fixing the position of torn off part of the limb sleeve on the arm when the desired size for the accessible area is reached.

13. The method of claim 12, wherein the fixing of the torn off part of the limb sleeve is executed by applying a tape on the torn off part of the limb sleeve.
